# EUROPEAN PATENT APPLICATION

(11) **EP 3 607 989 A1**
(43) Date of publication of application: **12.02.2020**
(21) Application number: 17904780.8
(22) Date of filing: 06.04.2017
(51) Int. Cl.: A61M 25/00

(54) **TUBULAR BODY AND TUBULAR BODY HAVING CATHETER**

(71) Applicant: Asahi Intecc Co., Ltd., Seto-shi, Aichi 489-0071 (JP)
(72) Inventor: KIZUKA Takeshi, Nagoya-shi Aichi 463-0024 (JP)
(74) Representative: Winter, Brandl, Fürniss, Hübner, Röss, Kaiser, Polte - Partnerschaft mbB
(86) International application number: PCT/JP2017/014407
(87) International publication number: WO 2018/185917

(57) **Abstract**

Provided is a catheter which is excellent in flexibility to follow a guidewire and is not easily kinked even when the catheter is bent strongly. A catheter shaft 3 comprises a hollow inner layer 2 and an outer layer 5, and a wire 7a wound to form a hollow screw shape within the hollow inner layer 2 and the outer layer 5, wherein the wire 7a is slidable with respect to the inner layer 2 and the outer layer 5.

## Description

### BACKGROUND OF THE INVENTION

### Field of the Invention

The present invention relates to a tube and a catheter comprising the tube.

### Description of the Related Art

Stricture sites or obliteration sites formed in internal lumina such as blood vessels, bile ducts, or pancreatic ducts inhibit the proper flow of blood, bile, pancreatic juice, and the like in internal lumina. To ameliorate such flow, methods for treating stricture sites or obliteration sites using catheters have been widely performed.

In general, internal lumina such as blood vessels, bile ducts, and pancreatic ducts taper toward the ends such that their lumina become thinner, and are strongly curved. Accordingly, when a stricture site or an obliteration site is formed at the end of an internal lumen, a guidewire that is inserted in advance is strongly bent along the internal lumen. Moreover, a catheter to be used subsequently is required to have flexibility sufficient to follow the strongly bent guidewire and kink resistance to prevent kinking even when strongly bent.

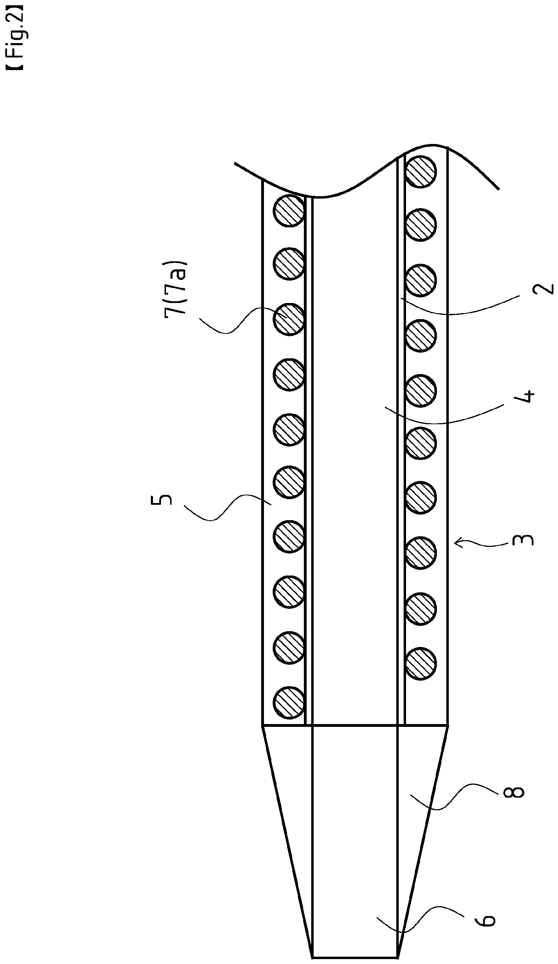

Conventionally, for example, Patent Literature 1 discloses a catheter 1 comprising an inner layer 7, a coil 3 wound around the outer circumference of the inner layer 7, and an outer layer 8 coating the outer circumference of the coil 3 (see Fig. 2 etc.).

Furthermore, Patent Literature 2 discloses a catheter 1 comprising an inner layer 13, multiple coils 111 to 114 which are wound around the outer circumference of the inner layer 13 and arranged longitudinally, and an outer layer 13 coating the outer circumference of the coils 111 to 114 coil (see Fig. 2 etc.).

However, in the catheter described in Patent Literature 1, the coil (hereinafter, described as "coil body") is completely fixed over the entire length between the inner layer and the outer layer. Therefore, the catheter is problematic in that when it is bent, a wire(s) forming the coil body is unable to be moved along the bending shape, and thus has poor flexibility to follow the guidewire.

Furthermore, the catheter described in Patent Literature 2 is also problematic in that: since multiple coil bodies are arranged but completely fixed between the inner layer and the outer layer, wires forming the coil bodies are unable to be moved according to the bending shape when the catheter is bent, resulting in poor flexibility to follow the guidewire.

### Citation List

### Patent Literature

Patent Literature 1: Japanese Unexamined Patent Publication No. 2001-218851
Patent Literature 2: Japanese Unexamined Patent Publication No. 2013-165926

### SUMMARY OF THE INVENTION

### Technical Problem

The present invention has been achieved to solve such problems, and an object of the present invention is to provide a catheter which is excellent in flexibility to follow a guidewire, and is not easily kinked even when the catheter is strongly bent.

### Solution to Problem

To achieve the object, a 1^{st} aspect of the present invention is a tube comprising a hollow resin body and a wire wound to form a hollow screw shape within the hollow resin body, wherein the wire is slidable with respect to the resin body.

Further, a 2^{nd} aspect of the present invention is a tube formed by winding a resin-coated wire consisting of a wire and a resin body covering the wire in a hollow screw shape, wherein the adjacent turns of the resin-coated wire are fixed, and the wire is slidable with respect to the resin body.

Further, a 3^{rd} aspect of the present invention is that, in the 1^{st} aspect of the present invention or the 2^{nd} aspect of the present invention, the wire forms gaps between the wire and the resin body.

Further, a 4^{th} aspect of the present invention is that, in any one of the 1^{st} aspect of the present invention to the 3^{rd} aspect of the present invention, the wire is fixed to the proximal end section of the resin body such that the distal end section of the resin body has gaps.

Furthermore, a 5^{th} aspect of the present invention is a catheter comprising the tube according to any one of the 1^{st} aspect of the present invention to the 4^{th} aspect of the present invention, a tip end connected to the distal end of the tube, and a connector connected to the proximal end of the tube.

### Advantageous Effects of Invention

According to the 1^{st} aspect of the present invention, in the tube comprising the hollow resin body and the wire wound to form a hollow screw shape within the hollow resin body, the wire is slidable with respect to the resin body, so as to be able to prevent the kinking of the tube and improve the flexibility of the tube.

Further, according to the 2^{nd} aspect of the present invention, in the tube formed by winding the resin-coated wire composed of the wire and the resin body covering the wire in a hollow screw shape, the adjacent turns of the resin-coated wire are fixed and the wire is slidable with respect to the resin body, so as to be able to prevent the kinking of the tube and further improve the flexibility of the tube.

Further, according to the 3^{rd} aspect of the present invention, in the 1^{st} aspect of the invention or the 2^{nd} aspect of the invention, the wire forms gaps between the wire and the resin body, so as to be able to further improve the flexibility of the tube in addition to exertion of the effect of the 1^{st} aspect of the invention or the 2^{nd} aspect of the invention.

Further, according to the 4^{th} aspect of the present invention, in any one of the 1^{st} aspect of the invention to the 3^{rd} aspect of the invention, the wire is fixed to the proximal end section of the resin body such that the distal end section of the resin body has gaps, so as to be able to further improve the flexibility of the distal end of the tube in addition to exertion of the effect of any one of the 1^{st} aspect of the invention to the 3^{rd} aspect of the invention.

Further, according to the 5^{th} aspect of the present invention, the catheter comprises the tube according to any one of the 1^{st} aspect of the invention to the 4^{th} aspect of the invention, the tip end connected to the distal end of the tube, and the connector connected to the proximal end of the tube, so as to be able to prevent kinking and improve flexibility to follow the guidewire.

### BRIEF DESCRIPTION OF THE DRAWINGS

Figure 1 is a general view of the catheter (tube) of a 1^{st} embodiment of the present invention.
Figure 2 is an enlarged sectional view of part A in Fig. 1.
Figure 3 shows a B-B cross section in Fig. 1.
Figure 4 is a sectional view of a portion of the catheter (tube) of a 2^{nd} embodiment, corresponding to that in Fig. 2.
Figure 5 is a general view of the catheter (tube) of a 3^{rd} embodiment.
Figure 6 is an enlarged view of a portion of part C in Fig. 5.
Figure 7 is an enlarged sectional view of part C in Fig. 5.
Figure 8 shows a D-D cross section in Fig. 5.
Figure 9 is a sectional view of a portion of the catheter (tube) of a 4^{th} embodiment, corresponding to that in Fig. 2.
Figure 10 is a sectional view of a portion of the catheter (tube) of a 5^{th} embodiment, corresponding to that in Fig. 2.

### DETAILED DESCRIPTION OF THE PREFERRED EMBODIMENTS

Hereinafter, embodiments of the present invention are described with reference to the drawings.

### <1^{st} embodiment>

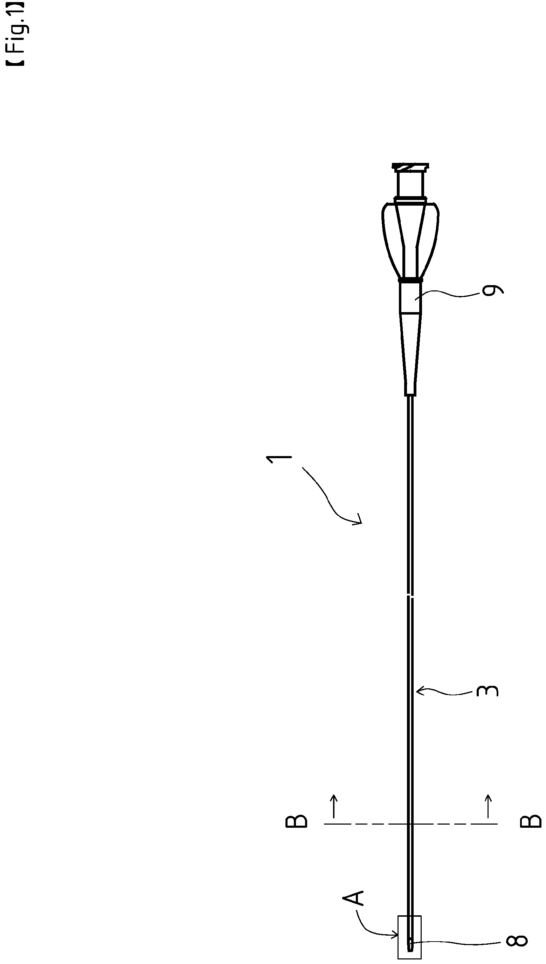

First, the catheter of the 1^{st} embodiment of the present invention is described with reference to Fig. 1 to Fig. 3.

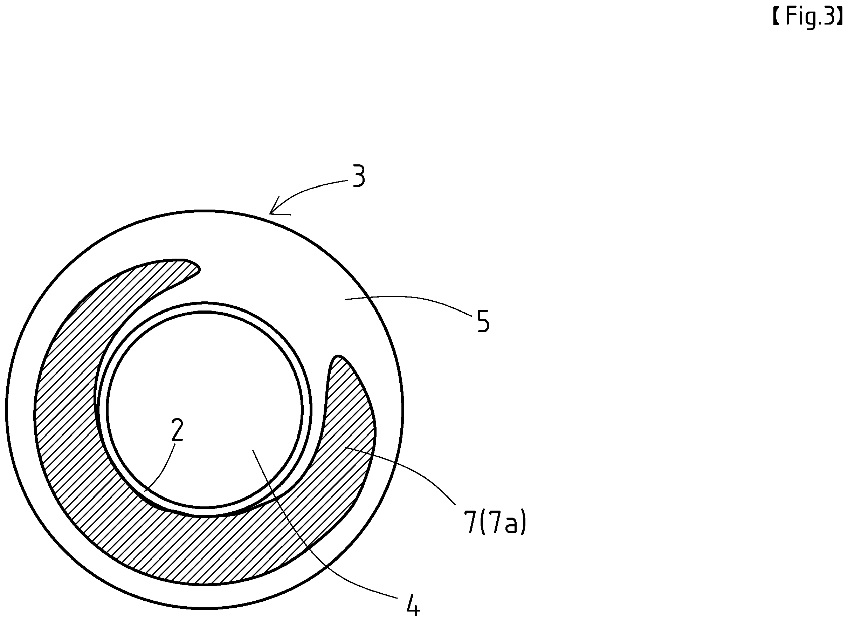

Fig. 1 is a general view of the catheter (tube) of the 1^{st} embodiment of the present invention. Fig. 2 is an enlarged sectional view of part A in Fig. 1. Fig. 3 shows a B-B cross section in Fig. 1.

In Fig. 1 and Fig. 2, the left side in each figure is a tip side (distal end side) to be inserted into a body, and the right side in each figure is the rear end side (proximal end side) to be operated by a clinician such as a doctor.

In Fig. 1, a catheter 1 is inserted into an internal lumen such as a blood vessel, a bile duct, or a pancreatic duct for treatment and diagnosis of a patient, and comprises a catheter shaft 3 (corresponding to the "tube" of the present invention), a tip end 8 connected to the distal end of the catheter shaft 3, and a connector 9 connected to the proximal end of the catheter shaft 3.

The catheter shaft 3 forms a hollow cylindrical shape and has, as shown in Fig. 2, sequentially in a radial direction from inside, an inner layer 2, a coil body 7 formed of a wire spirally wound around the outer circumference of the inner layer 2, and an outer layer 5 coating the outer circumference of the coil body 7.

The inner layer 2 is a hollow tube made of a resin, within which a lumen 4 is formed for insertion of a guidewire or another catheter thereinto. A resin material constituting the inner layer 2 is not particularly limited, and PTFE (polytetrafluoroethylene) is used in this embodiment.

The coil body 7 is constituted by winding a single wire 7a spirally around the outer circumference of the inner layer 2 such that adjacent wire 7a turns are spaced away from each other. In this embodiment, stainless steel (SUS304) is used as a material of the wire 7a constituting the coil body 7, but the material is not limited thereto. As the material of the wire 7a constituting the coil body 7, for example, a metal material such as tungsten or a Ni-Ti alloy may be used and a resin material such as reinforced plastic (PEEK) may also be used.

Note that, in this embodiment, the wire 7a is constituted of a single wire, however, the wire 7a may be constituted of not only a single wire, but also multiple wires. However, the wire 7a constituted of a single wire can further improve flexibility.

The outer layer 5 is made of a resin and coats the outer circumference of the inner layer 2 and the coil body 7 to form a tube. A resin material for constituting the outer layer 5 is not particularly limited. In this embodiment, polyamide, polyamide elastomer, polyester, polyurethane and the like are used.

In this embodiment, the wire 7a constituting the coil body 7 is arranged to be slidable with respect to the outer layer 5. Specifically, the wire 7a is spirally wound around the outer circumference of the inner layer 2 without being fixed to the inner layer 2 and the outer layer 5, and thus constituted so that it is spirally slidable.

Further, the tip end 8 made of a resin is connected to the distal end of the catheter shaft 3. The tip end 8 is a hollow tapered member having a lumen 6 communicating with a lumen 4. A resin material constituting the tip end 8 is not particularly limited. In this embodiment, polyurethane, polyurethane elastomer, or the like is used.

Further, the tip end 8 may contain radiation-impermeable powder in the resin. For example, the tip end 8 contains, in the resin, radiation-impermeable powder (e.g., tungsten powder) ranging from about 65 w% to about 90w%, so that a clinician such as a doctor can precisely grasp the position of the catheter 1 upon imaging.

Further, the connector 9 made of a resin is connected to the proximal end of the catheter shaft 3. The connector 9 is a hollow member having a lumen (not shown) communicating with the lumen 4. A resin material constituting the connector 9 is not particularly limited. In this embodiment, polycarbonate or the like is used.

According to the catheter shaft 3 or the catheter 1 of this embodiment, the wire 7a constituting the coil body 7 is arranged to be slidable with respect to the inner layer 2 and the outer layer 5, so as to be able to improve the flexibility of the catheter shaft 3 and the catheter 1 and prevent the kinking of the catheter shaft 3 and the catheter 1.

### <2^{nd} embodiment>

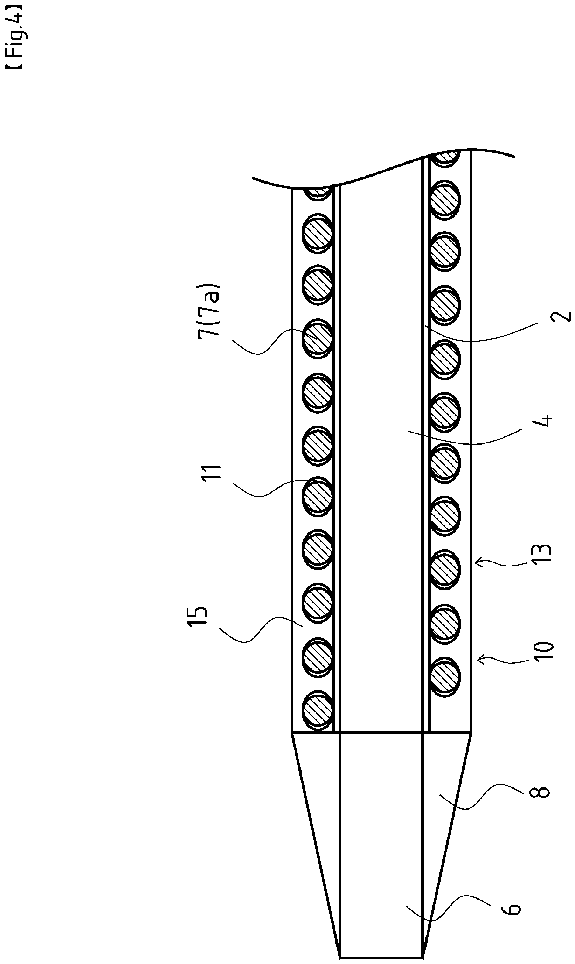

Next, the catheter of a 2^{nd} embodiment of the present invention is described with reference to Fig. 4. Fig. 4 is a sectional view of a portion of the catheter (tube) of the 2^{nd} embodiment, corresponding to that in Fig. 2.

Note that in the 2^{nd} embodiment, members same as the members constituting the catheter 1 of the 1^{st} embodiment are numbered with the same numbers and descriptions therefor are omitted.

Also in Fig. 4, the left side in the figure is the tip side (distal end side) to be inserted into a body, and the right side in the figure is the rear end side (proximal end side) to be operated by a clinician such as a doctor.

In Fig. 4, a catheter 10 comprises a catheter shaft 13 (corresponding to the "tube" of the present invention), a tip end 8 connected to the distal end of the catheter shaft 13, and a connector 9 connected to the proximal end of the catheter shaft 3.

The catheter shaft 13 has, as shown in Fig. 4, sequentially in a radial direction from inside, an inner layer 2, a coil body 7 formed of a wire spirally wound around the outer circumference of the inner layer 2, and an outer layer 15 coating the outer circumference of the coil body 7.

The outer layer 15 is made of a resin and coats the outer circumference of the inner layer 2 and the coil body 7 to form a tube. A resin material constituting the outer layer 15 is not particularly limited. In this embodiment, polyamide, polyamide elastomer, polyester, polyurethane or the like is used.

In this embodiment, the wire 7a constituting the coil body 7 is arranged in a manner similar to that in the 1^{st} embodiment, such that it is arranged to be slidable with respect to the outer layer 15. Specifically, the wire 7a is spirally wound around the outer circumference of the inner layer 2 without being fixed to the inner layer 2 and the outer layer 5, and thus constituted so that it is spirally slidable.

When the catheter 10 of this embodiment is compared with the catheter 1 of the 1^{st} embodiment, the catheter 10 of this embodiment differs from the catheter 1 of the 1^{st} embodiment in that the catheter 10 comprises gap portions 11 formed between the wire 7a constituting the coil body 7 and the outer layer 15, but the catheter 1 comprises no such gap portion 11 formed therein.

According to the catheter shaft 13 or the catheter 10 of this embodiment, the wire 7a constituting the coil body 7 is arranged to be slidable with respect to the inner layer 2 and the outer layer 15 and the gap portions 11 are formed between the wire 7a constituting the coil body 7 and the outer layer 15, so that the flexibility of the catheter shaft 13 and the catheter 10 can be further improved.

### <3^{rd} embodiment>

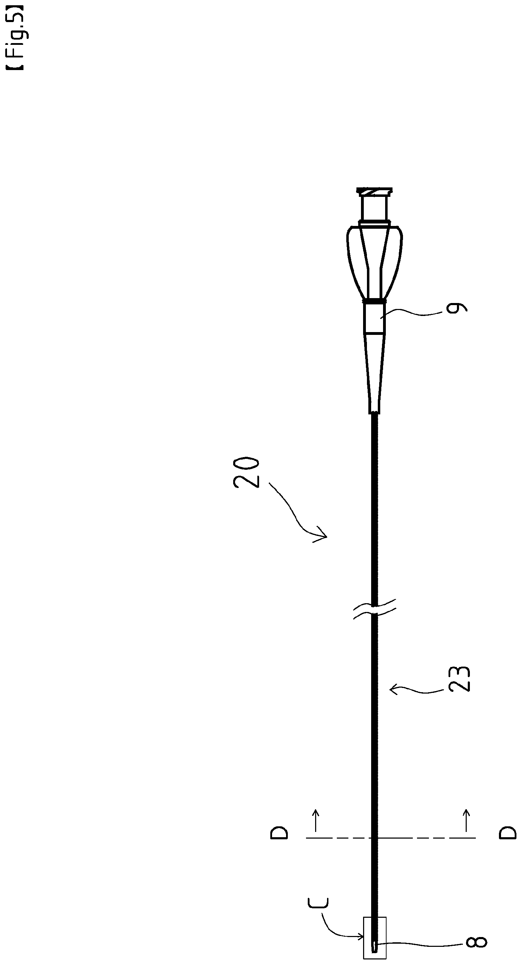

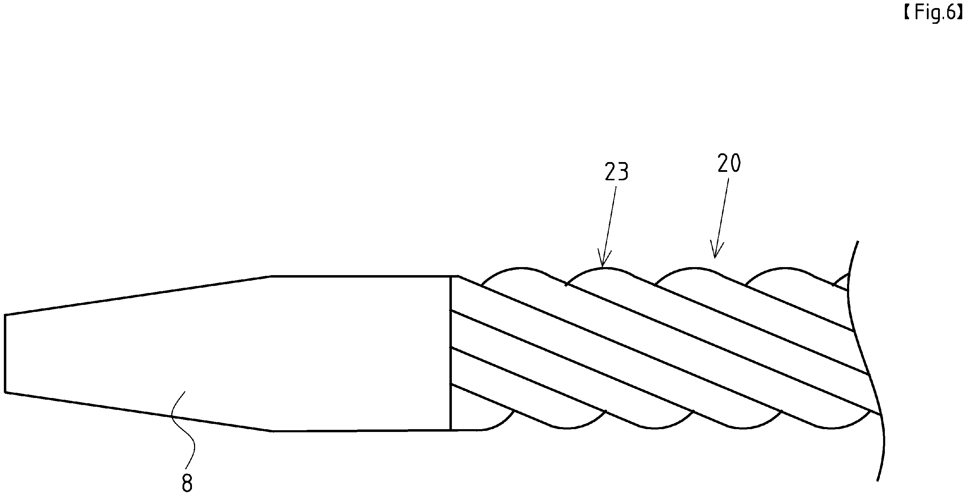

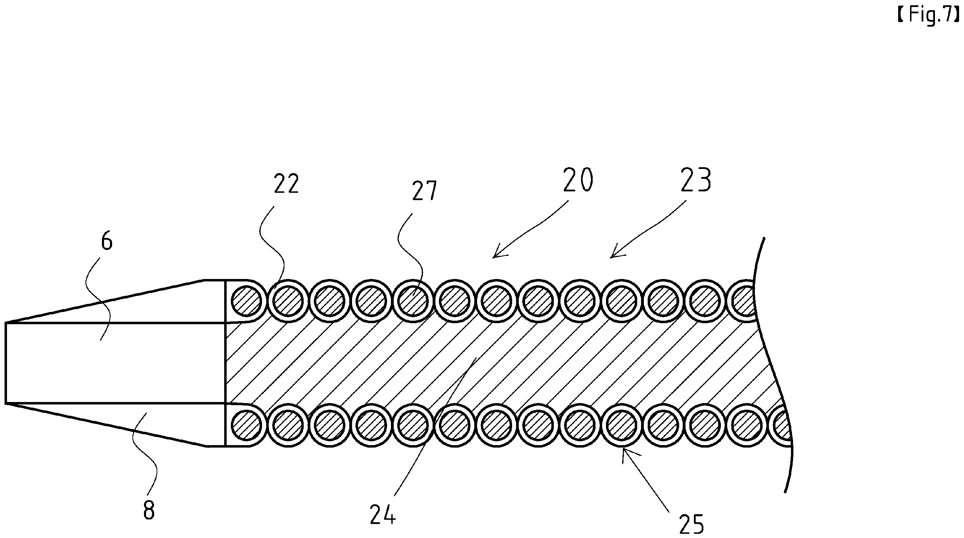

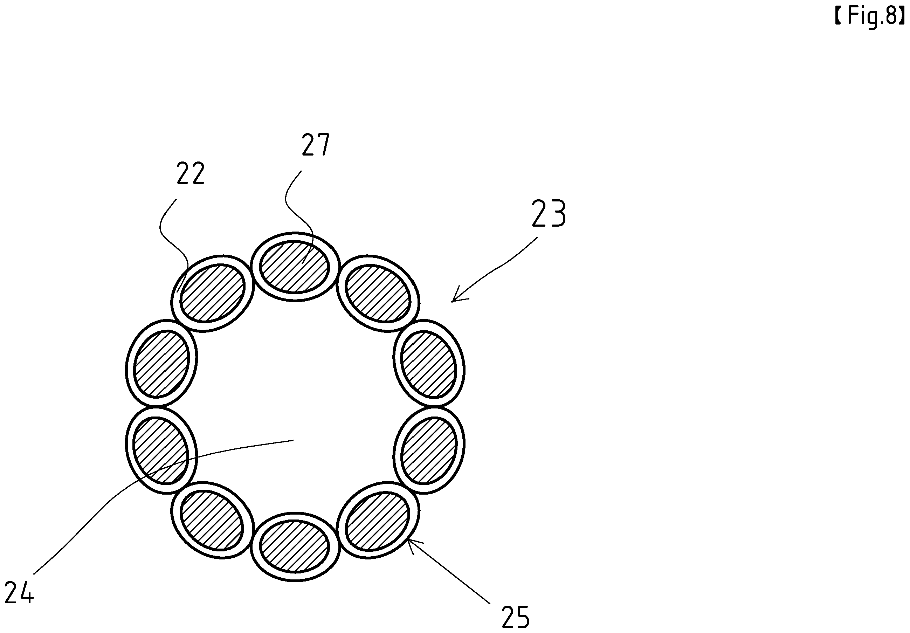

Next, the catheter of a 3^{rd} embodiment of the present invention is described with reference to Fig. 5 to Fig. 8. Fig. 5 is a general view of the catheter (tube) of the 3^{rd} embodiment. Fig. 6 is an enlarged view of a portion of part C in Fig. 5. Fig. 7 is an enlarged sectional view of part C in Fig. 5. Fig. 8 shows a D-D cross section in Fig. 5.

Note that in the 3^{rd} embodiment, members same as the members constituting the catheter 1 of the 1^{st} embodiment are numbered with the same numbers and descriptions therefor are omitted.

Also in Fig. 5 to Fig. 7, the left side in each figure is the tip side (distal end side) to be inserted into a body and the left side in each figure is the rear end side (proximal end side) to be operated by a clinician such as a doctor.

In Fig. 5, a catheter 20 comprises a catheter shaft 23 (corresponding to the "tube" of the present invention), a tip end 8 connected to the distal end of the catheter shaft 23, and a connector 9 connected to the proximal end of the catheter shaft 23.

The catheter shaft 23 is formed as shown in Fig. 6 to Fig. 8, wherein multiple resin-coated wires 25 (in this embodiment, ten wires), each comprising a wire 27 and a resin film 22 coating the outer circumference of the wire 27, are spirally wound to form a hollow screw shape. Here, the adjacent turns of the resin-coated wires 25 are characterized in that contact portions between the resin films 22 of the resin-coated wires 25 are each spirally welded. The catheter shaft 23 constitutes a hollow tube as a whole. Moreover, the catheter shaft 23 forms a lumen 24 with an irregular surface for insertion of a guidewire or another catheter thereinto.

The wires 27 of this embodiment are slidable with respect to the resin films 22. Specifically, the wires 27 are spirally wound together with the resin films 22 without being fixed to the resin films 22, and thus constituted so that they are spirally slidable.

A resin material constituting the resin films 22 is not particularly limited. In this embodiment, polyamide, polyamide elastomer, polyester, polyurethane, PTFE (polytetrafluoroethylene) or the like is used.

Note that in this embodiment, the catheter shaft 23 is constituted of multiple resin-coated wires 25 but may also be constituted of a single resin-coated wire 25. The catheter shaft 23 constituted of a single resin-coated wire can further improve flexibility.

Further, in this embodiment, the catheter shaft 23 is constituted of ten resin-coated wires 25. However, the number of the wires 25 is not limited to ten and may be any number. However, the catheter shaft 23 constituted of a smaller number of resin-coated wires is preferable in view of flexibility.

Further, the tip end 8 made of a resin is connected to the distal end of the catheter shaft 23. The tip end 8 is a hollow tapered member having a lumen 6 communicating with a lumen 24.

Moreover, the connector 9 made of a resin is connected to the proximal end of the catheter shaft 23. The connector 9 is a hollow member having a lumen (not shown) communicating with the lumen 24.

According to the catheter shaft 23 or the catheter 20 of this embodiment, the wires 27 are arranged to be slidable with respect to the resin films 22, so that the flexibility of the catheter shaft 23 and that of the catheter 20 can be improved and the kinking of the catheter shaft 23 and the catheter 20 can be prevented.

### <4^{th} embodiment>

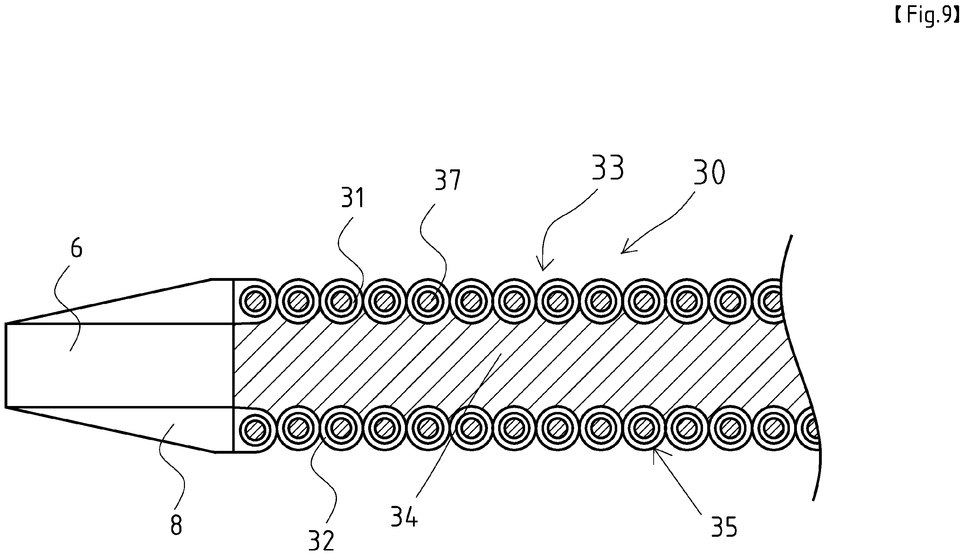

Next, the catheter of the 4^{th} embodiment of the present invention is described with reference to Fig. 9.

Fig. 9 is a sectional view of a portion of the catheter (tube) of the 4^{th} embodiment, corresponding to that in Fig. 2.

Note that in the 4^{th} embodiment, members same as the members constituting the catheter 20 of the 3^{rd} embodiment are numbered with the same numbers and descriptions therefor are omitted.

Also in Fig. 9, the left side in the figure is a tip side (distal end side) to be inserted into a body, and the right side in the figure is the rear end side (proximal end side) to be operated by a clinician such as a doctor.

In Fig. 9, a catheter 30 comprises a catheter shaft 33 (corresponding to the "tube" of the present invention), a tip end 8 connected to the distal end of the catheter shaft 33, and a connector 9 connected to the proximal end of the catheter shaft 33.

The catheter shaft 33 is formed as shown in Fig. 9, wherein multiple resin-coated wires 35 (in this embodiment, ten wires), each comprising a wire 37 and a resin film 32 coating the outer circumference of the wire 37, are spirally wound to form a hollow screw shape. Here, the adjacent turns of the resin-coated wires 35 are characterized in that contact portions between the resin films 32 of the resin-coated wires 35 are each spirally welded. The catheter shaft 33 constitutes a hollow tube as a whole. Moreover, the catheter shaft 33 forms a lumen 34 with an irregular surface for insertion of a guidewire or another catheter thereinto.

The wires 37 of this embodiment are slidable with respect to the resin films 32. Specifically, the wires 37 are spirally wound together with the resin films 32 without being fixed to the resin films 32, and thus constituted so that they are spirally slidable.

A resin material constituting the resin films 32 is not particularly limited. In this embodiment, polyamide, polyamide elastomer, polyester, polyurethane, PTFE (polytetrafluoroethylene) or the like is used.

Note that in this embodiment, the catheter shaft 33 is constituted of multiple resin-coated wires 35 but may also be constituted of a single resin-coated wire 35. The catheter shaft 33 constituted of a single resin-coated wire is preferable in view of flexibility.

Further, in this embodiment, the catheter shaft 33 is constituted of ten resin-coated wires 35. However, the number of the wires 35 is not limited to ten and may be any number of wires. However, the catheter shaft 33 constituted of a smaller number of resin-coated wires is preferable in view of flexibility.

Moreover, the tip end 8 made of a resin is connected to the distal end of the catheter shaft 33. The tip end 8 is a hollow tapered member having a lumen 6 communicating with a lumen 34.

The connector 9 made of a resin is connected to the proximal end of the catheter shaft 33. The connector 9 is a hollow member having a lumen (not shown) communicating with the lumen 34.

When the catheter 30 of this embodiment is compared with the catheter 20 of the 3^{rd} embodiment, the catheter 30 of this embodiment differs from the catheter 20 of the 3^{rd} embodiment in that the catheter 30 comprises gap portions 31 formed between the wires 37 and the resin films 32, but the catheter 20 comprises no such gap portion formed therein.

Therefore, according to the catheter shaft 33 or the catheter 30 of this embodiment, the wires 37 are arranged to be slidable with respect to the resin films 32, and the gap portions 31 are formed between the wires 37 and the resin films 32, so that flexibility of the catheter shaft 33 and the catheter 30 can further be improved.

### <5^{th} embodiment>

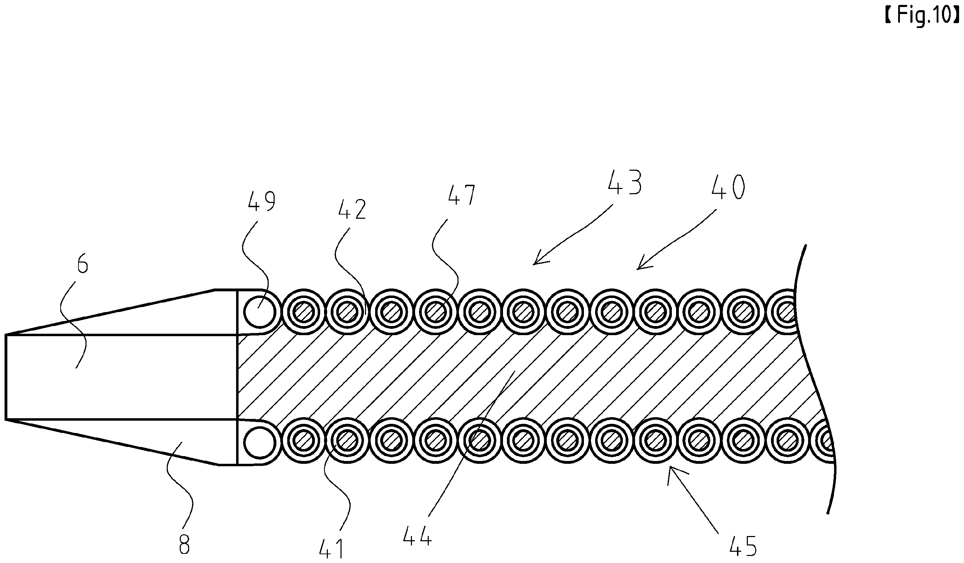

Finally, the catheter of the 5^{th} embodiment of the present invention is described with reference to Fig. 10. Fig. 10 is a sectional view of a portion of the catheter (tube) of the 5^{th} embodiment, corresponding to that in Fig. 2.

Note that in the 5^{th} embodiment, members same as the members constituting the catheter 20 of the 3^{rd} embodiment are numbered with the same numbers and descriptions therefor are omitted.

Also in Fig. 10, the left side in the figure is the tip side (distal end side) to be inserted into a body, and the right side in the figure is the rear end side (proximal end side) to be operated by a clinician such as a doctor.

In Fig. 10, a catheter 40 comprises a catheter shaft 43 (corresponding to the "tube" of the present invention), a tip end 8 connected to the tip of the catheter shaft 43, and a connector 9 connected to the base end of the catheter shaft 43.

The catheter shaft 43 is formed as shown in Fig. 10, wherein multiple resin-coated wires 45 (in this embodiment, ten wires), each comprising a wire 47 and a resin film 42 coating the outer circumference of the wire 47, are spirally wound to form a hollow screw shape. Here, the adjacent turns of the resin-coated wires 45 are characterized in that contact portions between the resin films 42 of the resin-coated wires 45 are each spirally welded. The catheter shaft 43 constitutes a hollow tube as a whole. The catheter shaft 43 forms a lumen 44 with an irregular surface for insertion of a guidewire or another catheter thereinto.

The wires 47 of this embodiment are slidable with respect to the resin films 42 at portions other than the proximal ends. Specifically, the wires 47 are spirally wound together with the resin films 42 while the portions other than the proximal ends of the wires 47 are not fixed to the resin films 42, and thus constituted so that they are spirally slidable.

Furthermore, the wires 47 of this embodiment form gap portions 41 between the wires 47 and the resin films 32. Therefore, the flexibility of the catheter shaft 43 and the catheter 40 can further be improved.

A resin material constituting the resin films 42 is not particularly limited. In this embodiment, polyamide, polyamide elastomer, polyester, polyurethane, PTFE (polytetrafluoroethylene) or the like is used.

Note that in this embodiment, the catheter shaft 43 is constituted of multiple resin-coated wires 45 but may also be constituted of a single resin-coated wire 45. The catheter shaft 43 constituted of a single resin-coated wire is preferable in view of flexibility.

Further, in this embodiment, the catheter shaft 43 is constituted of ten resin-coated wires 45. However, the number of the wires 45 is not limited to ten and may be any number of wires. However, the catheter shaft 43 constituted of a smaller number of resin-coated wires is preferable in view of flexibility.

Moreover, the tip end 8 made of a resin is connected to the distal end of the catheter shaft 43. The tip end 8 is a hollow tapered member having a lumen 6 communicating with a lumen 44.

Moreover, the connector 9 made of a resin is connected to the proximal end of the catheter shaft 43. The connector 9 is a hollow member having a lumen (not shown) communicating with the lumen 44.

When the catheter 40 of this embodiment is compared with the catheter 30 of the 4^{th} embodiment, the catheter 40 of this embodiment differs from the catheter 30 of the 4^{th} embodiment in that the catheter 40 comprises the wires 47 having the proximal ends fixed to the resin films 42; and gap portions 49 formed in the distal end section of the resin films 42 where no wires 47 are present, though the catheter 30 comprises no such gap portion formed therein.

Therefore, according to the catheter shaft 43 or the catheter 40 of this embodiment, the wires 47 are arranged to be slidable with respect to the resin films 42, the gap portions 41 are formed between the wires 47 and the resin films 32, and gap portions 49 are formed in the distal end section of the resin films 42 where no wires 47 are present, so that the flexibility of the catheter shaft 43 and the catheter 40, and particularly the flexibility at the distal end section can further be improved.

### Reference Signs List

- 1, 10, 20, 30, 40: Catheter
- 2: Inner layer
- 3, 13, 23, 33, 43: Catheter shaft (tube)
- 4, 6, 24, 34, 44: Lumen
- 5, 15: Outer layer
- 7: Coil
- 7a, 27, 37: Wire
- 8: Tip end
- 9: Connector
- 11, 31, 41, 49: Gap portion
- 22, 32, 42: Resin film
- 25, 35, 45: Resin-coated wire

## Claims

1. A tube comprising a hollow resin body and a wire wound to form a hollow screw shape within the hollow resin body, wherein the wire is slidable with respect to the resin body.

2. A tube formed by winding a resin-coated wire consisting of a wire and a resin body covering the wire in a hollow screw shape, wherein
adjacent turns of the resin-coated wire are fixed, and
the wire is slidable with respect to the resin body.

3. The tube according to claim 1 or 2, wherein the wire forms gaps between the wire and the resin body.

4. The tube according to any one of claims 1 to 3, wherein the wire is fixed to a proximal end section of the resin body such that gaps are present in a distal end section of the resin body.

5. A catheter, comprising
the tube according to any one of claims 1 to 4,
a tip end connected to a distal end of the tube, and
a connector connected to a proximal end of the tube.
